# EUROPEAN PATENT SPECIFICATION

(11) **EP 2 175 901 B1**
(45) Date of publication and mention of the grant of the patent: **28.03.2012**
(21) Application number: 08745217.3
(22) Date of filing: 07.04.2008
(51) Int. Cl.: A61L 31/02, A61L 31/14, A61F 2/82

(54) **THIN FILM TISSUE REPAIR MATRIX**
DÜNNFILM-GEWEBEREPARATUR-MATRIX
MATRICE DE RÉPARATION TISSULAIRE EN FILM MINCE

(30) Priority: 05.04.2007 US 697151
(43) Date of publication of application: 21.04.2010
(73) Proprietor: Cordis Corporation, Miami Lakes, FL 33014 (US)
(72) Inventor: RUSSELL, Scott M., San Jose, California 95125 (US); LUEHRS, Kirsten, Palo Alto, California 94306 (US); NEDVETSKY, Alex, Dublin, California 94568 (US)
(74) Representative: Brown, George Laurence
(86) International application number: PCT/US2008/059542
(87) International publication number: WO 2008/124673

(56) References cited:
- EP-A- 1 506 747
- WO-A-00/04204
- WO-A-01/53559
- WO-A-98/40035
- WO-A-2004/028340
- RU-C2- 2 199 968
- US-A1- 2003 208 260

## Description

### FIELD OF THE INVENTION

The present invention relates to a thin film tissue repair matrix made from a super elastic material such as Nitinol for use within a patient's body or on as part of a skin graft onto a patient's body.

### BACKGROUND OF THE INVENTION BACKGROUND

A thin film tissue repair matrix is an implantable device used to provide structural support within a patient to help repair a patient's organs. These support device applications include: lung repair, pleurodesis, hernia repair, skin grafts, etc. The tissue repair matrixes must be flexible and have surface areas that are large enough to provide the necessary support for the internal organ. In order to provide a large flexible surface area, tissue repair matrix is frequently a mesh or woven structure.

A problem with the prior art tissue repair matrix is that they cannot be compressed for insertion through a small minimally invasive hole and then expanded within the patient prior to that can cause trauma within the patient. What is needed is a tissue repair matrix that can be compressed for insertion into a patient through a minimally invasive hole in the patient and then expanded for implantation within the patient.

WO-A-2004/028340 relates to vacuum deposited nitinol alloy films and thin film graft materials. A microporous thin film metal covering may be used with a structural support element, such as stent.

WO-A-01/53559 discusses a thin film stent formed of a sputtered nitinol shape memory alloy.

WO-A-00/04204 relates to a thin film stent. The stent is created by sputtering hot molten metal onto a mould substrate.

US-A-2003/0208260 discusses procedures for introducing stents and stent-grafts. A stent-graft may be folded for introduction.

WO-A-98/40035 relates to a flexible tissue supporting device which is a stent. The stent comprises continuous circumferential strips joined by wavy strips.

EP-A-1506747 discusses a stent with multiple anchors. A plurality of barbs face outwardly when the stent is expanded.

RU-C-2199968 relates to an obturator of a hernial hila. A reticular implant is made of nickelide-titaniferous ligature.

### SUMMARY OF THE INVENTION

A first aspect of the present invention is an improved tissue repair matrix for implantation into a patient as defined in appended claim 7. The tissue repair matrix is made of a thin film of super elastic alloy such as Nitinol that is formed by vapor deposition. The matrix is cut into a pattern that provides porosity and may have features that promote adhesion of the tissue. The repair matrix has a first smaller surface area in a compressed state which allows insertion into the patient through a minimally invasive hole. After the tissue repair matrix is inserted into the patient it is expanded to a larger surface area in an expanded state. The tissue repair matrix is implanted into the patient in the expanded form as a "bandage" support surface for body tissue. The porous matrix provides a surface on which new tissue grows in a damaged area to help the patient heal. Applications for the inventive repair matrix include: lung repair, pleurodesis, hernia repair and skin grafting.

The repair matrix may have a planar surface or a three dimensional surface. If the inventive tissue repair matrix is used as a planar member, the super elastic alloy is vapor deposited onto a planar substrate and machined with the desired repair matrix pattern. In a three dimensional embodiment, the repair matrix may deposited in the planar form and then converted into a three dimensional shape through a deformation and heat treating process. Alternatively, the super elastic alloy may also be vapor deposited onto a three dimensional substrate so that it does not require post deposition heat treatment to obtain the required shape. A three dimensional repair matrix may be desirable for specific medical applications. For example, in a lung repair application, the shape of the repair matrix may correspond to the surface of the lung that is being repaired.

The repair matrix has a plurality of hooks or barbs that provide anchors for the repair matrix to adhere to the organ being repaired. The hooks are sharp pointed features that are cut in the thin film of the repair matrix. In the first smaller compressed state, the hook may be flush with the thin film so the point of the hook is protected. This allows the repair matrix to be inserted into the patient without having any sharp points exposed. When the repair matrix expands to the second larger area, the hooks may bend away from the repair matrix. The bent hooks engage the organs of the patient and also provide a hole in the repair matrix for ingrowth.

The repair matrix may also include amorphic circles that are attached to the outer edges that make the inventive tissue repair matrix a-traumatic to the implanted patient. The amorphic circles may also have hooks (described above) and holes which allow for in-growth after the tissue repair matrix has been implanted within the patient. The holes in the amorphic circles can also be used as suture points that are used to secure the tissue repair matrix to the desired location within the patient.

The tissue repair matrix is cut into an intricate pattern of interconnected struts, loops and bridges. Bending of the struts, loops and bridges allows the tissue repair matrix to transform in area between a compressed state and an expanded state. After the tissue repair matrix is fabricated, it can be coated with polymers, therapeutic agents, bioactive materials or radio-opaque materials depending upon the application.

The tissue repair matrix is made from a "super elastic" metal alloy such as Nitinol. Super elastic metal alloys have the physical characteristics of being extremely elastic when cooled to the martensitic molecular phase. In this phase, the inventive tissue repair matrix can be compressed into a small volume without springing back to its expanded shape. In the compressed form, the gaps separating the struts, loops and bridges are very small so that the adjacent struts are in very close proximity to each other. In addition to being compressed in a planar manner, the tissue repair matrix may also be bent or rolled out of plane in an accordion manner to further compresses the tissue repair matrix.

Before the repair matrix is implanted in the desired area within the patient, it is heated to change the super elastic alloy to the austenitic phase causing the tissue repair matrix to expand. The final austenite transition temperature may be about 24° C to about 37° C so that exposure to the patient's body heat causes the desired transformation. In the expanded state, the struts, loops and bridges bend so the repair matrix expand to a larger area with larger gaps between the components. After the repair matrix is fully expanded, it has a sufficient area to provide support to the internal organs and can be attached to the patient through in-growth or sutures through the holes in the amorphic circles.

According to another aspect of the invention, there is provided a method for manufacturing a tissue repair matrix as defined in appended claim 1.

### BRIEF DESCRIPTION OF DRAWINGS

The foregoing and other aspects of the present invention will best be appreciated with reference to the detailed description of the invention in conjunction with the accompanying drawings, wherein:
FIG. 1 is a view of an embodiment of the tissue repair matrix in the compressed state.
FIG. 2 is a view of an embodiment of the tissue repair matrix in the compressed state.
FIG. 3 is an enlarged view of an amorphous circle that has a hook.
FIG. 4 is an enlarged view of the tissue repair matrix in the compressed state.
FIG. 5 is a side view of an embodiment of the tissue repair matrix folded out of plane.
FIG. 6 is an enlarged view of a portion of the tissue repair matrix in the expanded state.
FIG. 7 is an alternative embodiment of the tissue repair matrix.

### DETAILED DESCRIPTION

The present invention is directed towards a tissue repair matrix which is made from a thin film of super elastic alloy. Although the super elastic alloy is described as Nitinol (Ni-Ti alloy), other alloys with similar super elastic properties may be used. Very thin Nitinol film stock is commercially available from a number of suppliers including Nitinol Devices & Components, Fremont California.

Alternatively, the Nitinol thin film stock may be formed through a vapor deposition process. Vapor deposition, as used herein, refers to any process of depositing metals and metal compounds from a source to a substrate or target by dissipating metal ions from the source in a vaporous medium. Examples of vapor deposition processes that may be used to make the present invention include evaporation vapor deposition, sputtering deposition, chemical vapor deposition, etc.

In the evaporation vapor deposition process, vapor is generated by heating a source material to a temperature to cause the vaporization thereof. The evaporating metal atom leaves the surface of the Nitinol source material in a straight line. Therefore, the highest quality deposition layers are deposited when the source-to-substrate distance is less than the mean path distance between collisions of the vaporized metal and the surrounding vacuum chamber. The substrate may be rotated or translated during the evaporation process so that a uniform Nitinol layer is deposited on the substrate.

In the sputtering process, a source material is placed in a vacuum chamber with a substrate material. A radio-frequency power source gives the substrate a positive charge relative to the Nitinol source material. The source material is bombarded with inert gas ions from an ion beam or a plasma discharge to cause the source material to dislodge. These dislodged atoms are then deposited onto the substrate to form the thin film layer.

In the chemical vapor deposition process, reactant gases that may be diluted in a carrier gas are injected into a reaction chamber. The gas mixture is heated and the atoms are deposited on a substrate. The deposition continues until the desired thickness is formed. The thickness of the deposited Nitinol used to make the super elastic alloy tissue repair matrix may range from about 2.54µm to about 2.54mm (about 0.0001 to about 0.1 inch).

The thin film vapor deposited Nitinol can be planar or have a three dimensional shape. Thus, the substrate that the Nitinol is deposited on can be a planar or three-dimensional surface. In order to simplify the removal of the deposited Nitinol from the planar or three-dimensional substrate, a release layer may be applied to the substrate prior to the Nitinol vapor deposition.

The Nitinol sheet stock may be cut into the desired fully expanded tissue repair matrix pattern while in the austenitic phase or in the martensitic phase. The phase of the Nitinol material is temperature dependent. In general, the austenitic transition temperature Af is about 24° C to about 37° C. At temperatures above the austenitic transition temperature, the Nitinol will be in the Austenitic phase. At lower temperatures, the Nitinol may be fully or partially in the martensitic phase. If the tissue repair matrix is cut in the martensitic phase, it can then be maintained in the expanded shape while it is heat treated to convert the Nitinol to the austenitic phase. The tissue repair matrix can then be cooled to transform the tissue repair matrix to the martensitic phase before the tissue repair matrix is compressed into a compact state for implantation in a patient. The austenitic phase shape is the shape that the tissue repair matrix will attempt to assume whenever it is heated above the austenitic transition temperature.

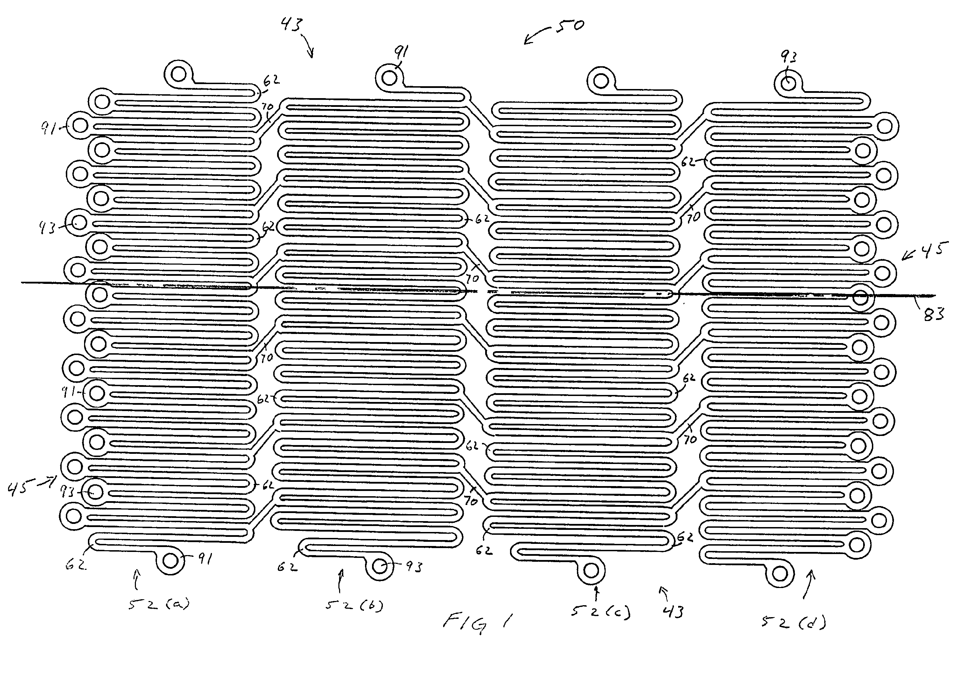

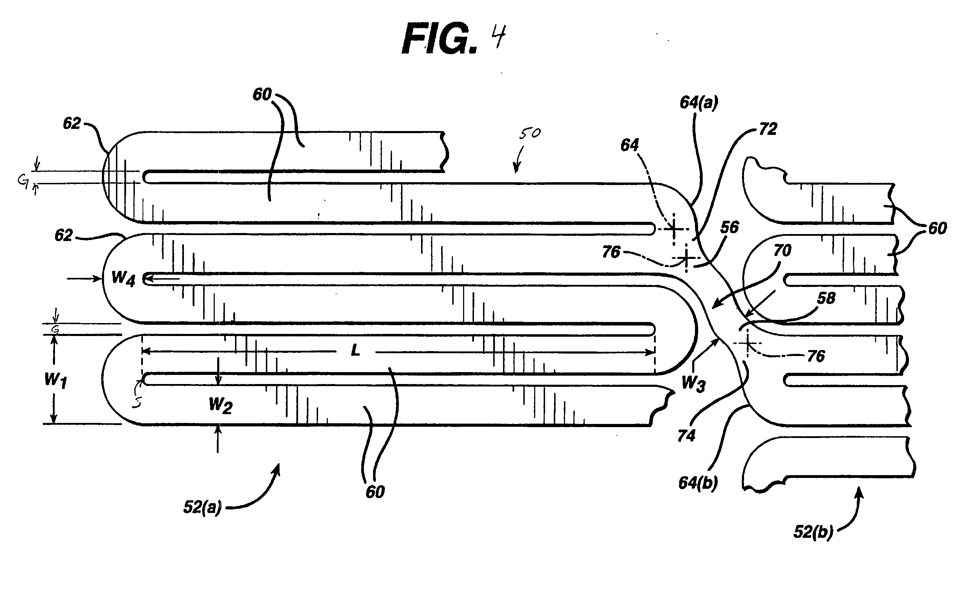

With reference to FIG. 1 and FIG. 4, the tissue repair matrix 50 is cut into an intricate pattern of adjacent planar strips 52(a)-52(d) that are connected by a plurality of bridges 70. The planar strips 52(a)-52(d) are each made of a plurality of interconnected struts 60 and loops 62. The lengths of the struts, loops and bridges may vary within each of the planar strips 52(a)-52(d). In an embodiment, the Nitinol sheet stock is loaded into a machine that cuts the predetermined pattern of the expandable tissue repair matrix. Machines that can cut sheets of Nitinol are well known to those of ordinary skill in the art and are commercially available. During this machining process, the metal sheet is typically held stationary while a cutting tool, preferably under microprocessor control, moves over the sheet and cuts the desired tissue repair matrix pattern. The pattern dimensions and styles, laser positioning requirements, and other information are programmed into a microprocessor which controls all aspects of the process. The cutting tool can be a laser, laser chemical etch, water jet, electrical discharge machining, etc.

In an embodiment, a photochemical etch process may be used to cut the desired pattern into the Nitinol sheet. This process can include various process steps that are generally known as photolithography. A photoresist layer is deposited onto the Nitinol sheet and exposing the photosensitive layer to a pattern of light that matches the desired pattern that the sheet is to be cut into. The light chemically alters the exposed areas of the photoresist layer and a chemical reaction is used to remove the portions of the photosensitive layer that were not exposed to light. An etch process then cuts through the areas of the Nitinol that are not covered by the photoresist to form the patterned tissue repair matrix. The remaining photoresist is removed to produce the finished patterned tissue repair matrix.

In an embodiment, the tissue repair matrix may be made from a plurality of adjacent elongated planar strips 52(a)-52(d) that are secured adjacent to each other across the length of the tissue repair matrix 50 by a plurality of bridges 70. Although four adjacent elongated strips are shown, tissue repair matrixes with any number of elongated strips can be made. The elongated strips 52 each include a plurality of longitudinal struts 60 and a plurality of loops 62 that connect the adjacent struts 60. The adjacent struts 60 are connected with loops 62 in an alternating pattern at opposite ends of the struts 62, forming a serpentine or "S" shaped pattern. In the compressed state, the loops 62 are substantially semi-circular and appear to be about a 180° bend. The space between the struts 60 is very small because the 180° bends of the loops 62 cause the struts 60 to be compressed close to each other.

The tissue repair matrix may also includes a plurality of amorphic circles 91 that are attached along the outer edges of the tissue repair matrix 50. Along the short sides of the tissue repair matrix 50, the amorphic circles 91 are attached to the loops 62. Along the long sides of the tissue repair matrix 50, the amorphic circles 91 are attached at a terminal point along the longitudinal length of the outermost strut in the planar strip 52(a) - 52(d). The rounded surfaces of the amorphic circles 91 and loops 62 eliminate any sharp external features and make the tissue repair matrix 50 a-traumatic when implanted into a patient. The amorphic circles 91 are round features that may also have a smooth rounded edge, such as a "bull-nose" edge to further remove any sharp surfaces. The amorphic circles 91 will preferably have a rounded ring shape rather than a cylindrical shape with sharp edges. Because the repair matrix is very thin, this edge rounding may not be noticeable or necessary.

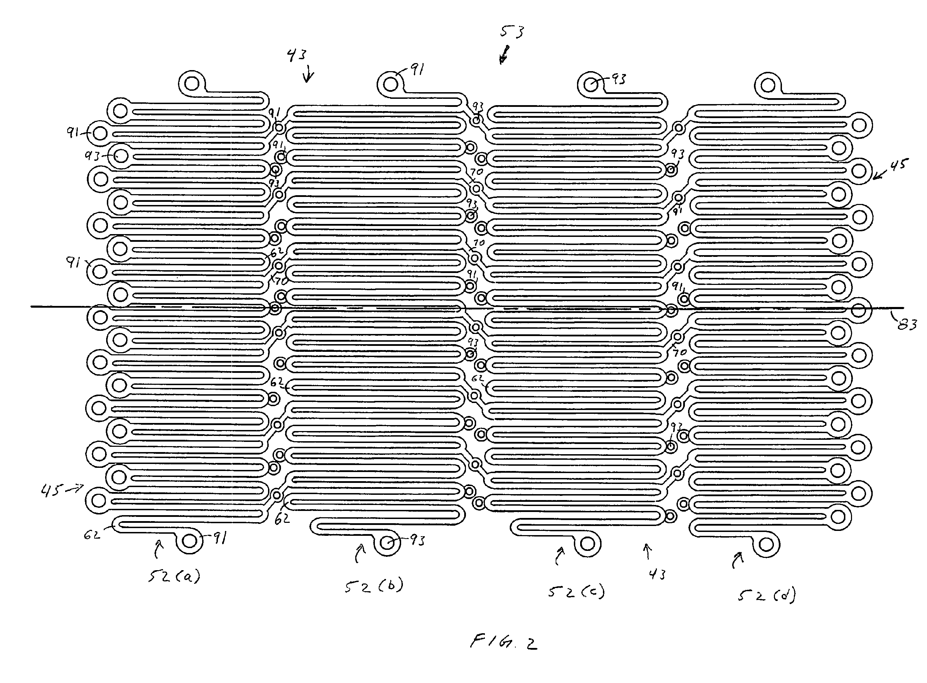

Holes 93 in the amorphic circles 91 provide areas for ingrowth to stabilize the tissue repair matrix 50 implanted into a patient. Alternatively, the holes 93 in the amorphic circles 91 may also be used to suture the tissue repair matrix 50 to tissue within the patient. The tissue repair matrix may be used as a physical graft structure or to provide physical support to organs within a patient. The amorphic circles 91 can range in diameters from about 25.4 µm to about 6.35 mm (about 0.001 to about 0.250 inch). The holes 93 are concentric with the amorphic circles 91 and may be proportional in diameter. The diameters of the holes 93 may range from about 10% to about 90% of the diameter of the amorphic circle 91. Although the amorphic circles 91 and holes 93 are illustrated only around the perimeter, the amorphic circles 91 can also be attached to any interior loop 62 of the tissue repair matrix 51 as shown in FIG. 2.

FIG. 2 illustrates an embodiment of the tissue repair matrix 53 having amorphic circles 91 and holes 93 placed in the bridges 70 and loops between the planar strips 52(a) - 52(d). This interior amorphic circles 91 and holes 93 provide additional areas for ingrowth and suture points to secure the tissue repair matrix within the patient. The internal amorphic circles 91 also allow the tissue repair matrix 53 to be cut to a size that is appropriate for the application. The bridges 70 can be cut to remove one or more of the elongated strips 52(a) - 52(d) from the tissue repair matrix 53. By cutting the bridges 70 next to the amorphic circles 91, the cut tissue repair matrix 53 has amorphic circles 91 that allows the cut edge to be sutured within the patient. The cut bridges 70 may need to be further smoothed after being cut to remove any sharp surfaces.

In alternative embodiments, some of the amorphic circles 91 may be replaced with rounded structures that provide the same a-traumatic edges to the tissue repair matrix 50 as the amorphic circles 91. These rounded structures may be spheres, ovals, rounded rectangles, rounded triangles, a rounded "T" end, etc. Like the amorphic circles 91, these rounded structures can be attached to the loops 62 anywhere in the tissue repair matrix 50, within any of the bridges 70 or at the struts 60 at the ends of the elongated strips 52(a) - 52(d). The rounded structures may also have holes formed through their centers for ingrowth or sutures.

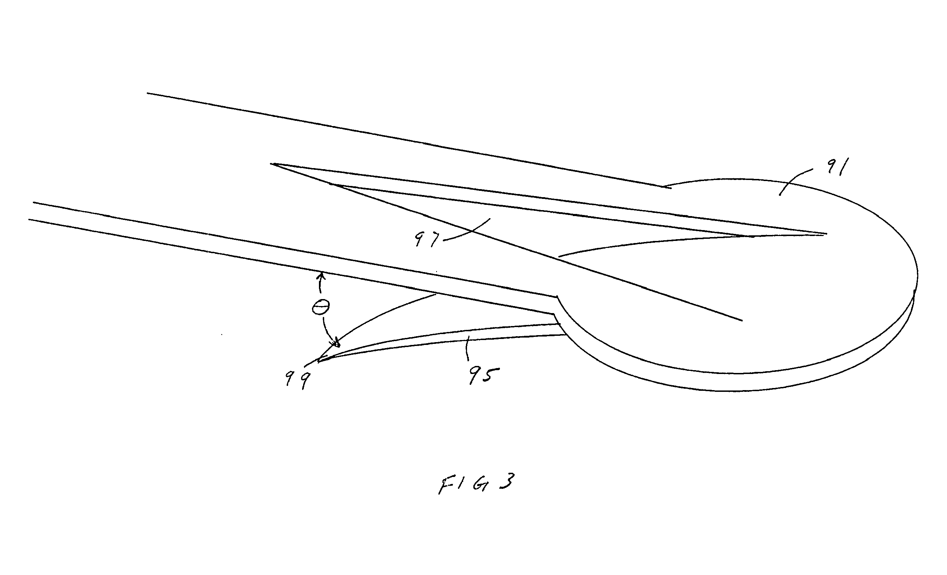

The repair matrix includes various mechanisms such as barbs and holes that are used to improve the adhesion of the repair matrix to the patient. The barbs and holes may be placed on any portion of the inventive repair matrix including the struts, loops, bridges and amorphic circles. With reference to FIG. 3, details of the hook 95 and hole 97 are shown on an amorphic circle 91. The hook 95 and hole 97 are formed by cutting the outer edges of the hook through the thin film. The hook 95 is then bent away at an angle θ of about 10° to about 90° so that it protrudes away from the plane of the repair matrix. The hook 95 may be bent in a gradual curve or sharply at the junction with the thin film. The tip 99 of the hook 95 may protrude between about 0.254mm and about 2.54mm (about 0.01 and about 0.1 inch) away from the planar surface and may be sharp. The hook 95 is designed to adhere to an organ surface and hold it in place while not being deep enough to cause damage. Alternatively, a hook 95 can be fully cut out of the repair matrix so that only a triangular hole 97 remains in the thin film.

In the preferred embodiment, the hook 95 is cut during the fabrication of the repair matrix and heat treated as described above so that it assumes the bent shape in the expanded austenitic phase. As discussed above, the repair matrix is cooled to a martensitic phase and compressed prior to implantation in the patient. In the compressed state, the hook 95 is deformed to be flush the hole 97. After the repair matrix is inserted into the patient and the phase changes to the austentic phase, the repair matrix assumes the expanded state and the hooks 95 bend away from the holes 97 to improve the adhesion to internal organs.

Although the bridges 70 appear to be straight structures connected to loops 62 on adjacent planar strips at an angle as shown in FIGS. 1 and 2, the bridges 70 may be curved to improve the structural performance of the inventive tissue repair matrix 50. The bridges 70 can best be described by referring to FIG. 4 that is an enlarged view of a portion of an embodiment of the compressed tissue repair matrix 50. Each bridge 70 has ends 56 and 58. End 56 of bridge 70 is attached to one loop 62 at a bridge-to-loop connection point 72 on a first elongated strip 52(a) and another end 58 attached to another loop 62 at a bride-to-loop connection point 74 on an adjacent elongated strip 52(b). In this example, the end 56 of bridge 70 is connected to loop 64(a) at bridge-to-loop connection point 72 and end 58 is connected to loop 64(b) at bridge-to-loop connection point 74. The bridge-to-loop connection points 72, 74 are separated angularly with respect to the longitudinal axis 83 of the tissue repair matrix 50 and are not horizontally opposite from each other.

The geometry of the struts is also designed to better distribute strain throughout the tissue repair matrix and minimize the opening size between the struts, loops and bridges. The number of struts, loops and bridges as well as the design of these components are important factors when determining the working properties and fatigue life properties of the tissue repair matrix. A tissue repair matrix that has a larger quantity of smaller sized struts per elongated strip improves the mechanical properties of the tissue repair matrix by providing greater rigidity than sheets made with fewer and larger struts. For example, a tissue repair matrix where the ratio of the number of struts per elongated strip to the strut length L (in inches) that is greater than 400 has increased rigidity (or greater than 157 with L in cm.).

After the tissue repair matrix is cut to the desired pattern, surface processing can be performed. The tissue repair matrix may be passivated by exposing the Nitinol to oxygen to form a layer of metal oxide which helps to prevent corrosion. The tissue repair matrix may also be polished to remove any rough surfaces through processes such as: mechanical polishing, electro polishing or chemical mechanical polishing. This polishing removes any sharp surfaces that may have been formed during the tissue repair matrix cutting processes.

Alternatively, the tissue repair matrix may be textured to improve the ingrowth after implantation or improve the adhesion of coatings applied to the tissue repair matrix. The texturing can be through photochemical etching, sand blasting, tumbling, etc. These textured surfaces can then be coated with different materials that will improve the implanted performance. These chemical coatings are generally intended to improve the biocompatibility of the tissue repair matrix within the patient's body by enhancing ingrowth, preventing rejection and resisting infection. These surface coatings include polymers, therapeutic agents and bioactive materials.

In an embodiment, some of the tissue repair matrix may also be coated with a radio-opaque material that is detectable with x-rays. The radio-opaque materials may alternatively be attached to the Nitinol tissue repair matrix by laser welding, adhesives, mechanical fasteners, etc. After the tissue repair matrix has been implanted within the patient, the implant area can be x-rayed to determine the exact position of the tissue repair matrix. If the tissue repair matrix is improperly positioned, the error can be detected and corrected.

After the tissue repair matrix 50 is cut and all surface coatings are applied, it is ready for use. The tissue repair matrix 50 is cooled below the martensitic transformation temperature to change the Nitinol to a super elastic material. The martensitic transformation temperature M_{f} may be between about 0° to about 15° C. In the martensitic phase, the interconnected struts 60, loops 62 and bridges 70 of the tissue repair matrix 50 can be compressed into a small area as shown in FIGS. 1-2. In the compressed shape, there may be very small gaps G between the adjacent struts 60 and loops 62. The compressed Nitinol alloy will remain in the compressed shape as long as the temperature remains below the austenitic transition temperature.

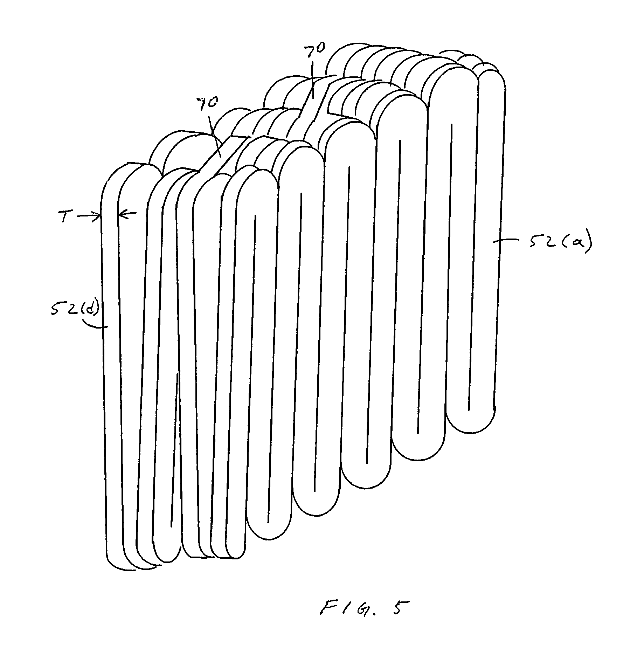

Although the tissue repair matrix 50 is shown in FIGS. 1-2 as being compressed in a planar configuration, it is also possible to further compress the tissue repair matrix 50 out of plane. FIG. 5 shows a side view of the tissue repair matrix 50 that is folded at the bridges 70 in an accordion manner. It is also possible to roll the medical sheet 50 in the compressed state. In the martensitic phase, the tissue repair matrix 50 will retain any of these out of plane compressed shapes until the phase of the metal is changed to the austenitic phase.

To implant the tissue repair matrix into a patient, the compressed repair matrix is held by a delivery apparatus and is inserted through a small incision cut through the skin of the patient. The repair matrix is then fully expanded before being permanently or temporarily implanted in the patient. The expansion of the tissue repair matrix inside the patient results from a molecular transformation of the metal alloy from the martensitic phase to the austenitic phase which results from the increased temperature inside the patient's body. The patient's body heat converts the phase of the Nitinol material into the austenitic phase. As the molecular structure of the metal alloy changes to the austenitic phase, the tissue repair matrix decompresses into its expanded shape.

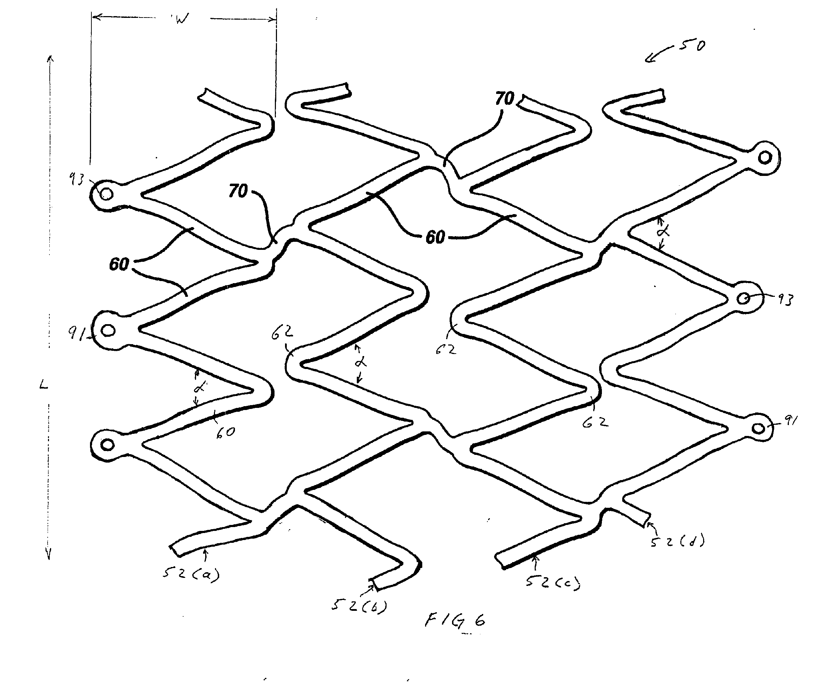

With reference to FIG. 6, a portion of the tissue repair matrix 51 is shown in the austenitic phase and expanded state. The expansion of the tissue repair matrix 51 may only be in line with the lengths of elongated strips 52(a) - 52(d). In the expanded state, the angle a between adjacent struts 60 connected by the loops 62 increases from a compressed angle of about 0° to about 5° to an expanded angle of about 30° to about 70°. The expanded angle α of the loops 62 causes the struts 60 to separate and causes the elongated strips 52(a) - 52(c) to expand in length. As the lengths of the strips expand, the widths of the elongated strips 52(a) - 52(c) get narrower because the struts 60 are angled across the width rather than running perpendicular across the widths. While the tissue repair matrix 51 is shown as being planar in the expanded state, it is possible to build a tissue repair matrix having a three dimensional shape in the expanded state and the compressed state as shown in FIG. 6.

After being fully expanded inside the patient, the tissue repair matrix 51 is positioned and secured in the patient using other medical instruments. The tissue repair matrix 51 may be attached within the patient's body by ingrowth through the holes 93 in the amorphic circles 91 and the gaps G between the struts 60, loops 62 and bridges 70. Alternatively, sutures may be sewn through the holes 93 to secure the tissue repair matrix 51 in place. After the tissue repair matrix 51 is implanted, all surgical tools are removed so the patient can heal.

As seen from FIGS. 1-5, the geometry of the tissue repair matrix changes significantly from the compressed state to its fully expanded state. As the tissue repair matrix expands, the strut angle α and strain levels in the struts, loops and bridges are affected. Preferably, all of the struts, loops and bridges will strain in a predictable manner so that the tissue repair matrix is structurally reliable and uniform in strength. In addition, it is preferable to minimize the maximum strain experienced by struts, loops and bridges, since Nitinol's mechanical strength properties are generally limited by strain rather than by stress like most metal materials. Most metals have a linear relationship between stress and strain in an elastic region and break after the stress exceeds the maximum tensile strength of the metal.

In contrast, when stress is applied to a specimen of a metal such as Nitinol exhibiting super elastic characteristics at a temperature above which the austenite is stable (i.e. the temperature at which the transformation of martensite phase to the austenite phase is complete), the specimen deforms elastically until it reaches a particular stress level where the alloy then undergoes a stress-induced phase transformation from the austenite phase to the martensite phase. As the phase transformation proceeds, the alloy undergoes significant increases in strain but with little or no corresponding increases in stress. The strain increases while the stress remains essentially constant until the transformation of the austenite phase to the martensite phase is complete. Thereafter, further increases in stress are necessary to cause further deformation. The martensitic metal first deforms elastically upon the application of additional stress and then plastically with permanent residual deformation.

If the load on the specimen is removed before any permanent deformation has occurred, the martensitic phase specimen will elastically recover and transform back to the austenite phase. The reduction in stress first causes a decrease in strain. As stress reduction reaches the level at which the martensite phase transforms back into the austenite phase, the stress level in the specimen will remain essentially constant (but substantially less than the constant stress level at which the austenite transforms to the martensite) until the transformation back to the austenite phase is complete, i.e. there is significant recovery in strain with only negligible corresponding stress reduction. The alloys are structurally stronger and more rigid in the austenitic phase than the martensitic phase. After the transformation back to austenite is complete, further stress reduction results in elastic strain reduction. This ability to incur significant strain at relatively constant stress upon the application of a load and to recover from the deformation upon the removal of the load is commonly referred to as super elasticity or "shape memory." See for example, U.S. Pat. No. 4,665,905 (Jervis) and U.S. Pat. No. 4,925,445 (Sakamoto et al.).

The transition between martensite and austenite phases can be controlled by the material temperature. The shape material is fully martensitic when it is colder than the final martensitic transition temperature M*_{f}* and fully austenitic when the material is heated above the final austenitic transition temperature A_{*f*.} The alloy may be partially martensitic and partially austenitic at temperatures between the final martensitic transition temperature M*_{f}* and the final austenitic transition temperature A*_{f}*. These shape memory alloys are stronger in the full austenitic phase than in the martensitic state, but no longer have the super elastic property. When a shape memory alloy structure is heated, it reverts, or attempts to revert, to its original heat-stable shape.

The super elastic metal alloys may comprise nickel, titanium and additional elements such as: niobium, hafnium, tantalum, tungsten and gold. The ratio of the nickel and titanium in the super elastic alloy will alter the martensite/austenite transition temperatures. An alloy having more than 50.5 atomic % nickel has a complete transition temperature from the martensite phase to the austenite phase (A_{f}) below human body temperature, so that austenite is the only stable phase at body temperature. The alloy preferably has an A_{f} in the range from about 24° C to about 37° C. The M_{f} is about 25 to 50 degrees C lower than the A_{f}.

Because these super elastic alloys are capable of extreme deformation, it is desirable to design products that will not exceed the maximum allowable strain during use. In trying to minimize the maximum strain experienced by the struts, loops and bridges, the present invention utilizes a structural geometry that distributes strain to areas of the tissue repair matrix which are less susceptible to failure. For example with reference to FIG. 4, one of the most vulnerable areas of the tissue repair matrix 50 is the inside surface S of the connecting loops 62 defined by the inner radius which undergoes the most deformation and therefore has the highest level of strain of all the tissue repair matrix features. This area is also critical in that it is usually compressed into the smallest radius on the tissue repair matrix. Stress concentrations are minimized by designing the loops 62 with the largest radii possible and/or have a smaller change in angle α between the compressed and fully expanded states shown in FIG. 6.

It is also desirable to minimize local strain concentrations on the bridge 70 and bridge connection points 72, 74. This can be accomplished at the outset through efficient utilization of materials in the struts 60, loops 62 and bridges 70 increases the strength and the ability of the inventive tissue repair matrix 50 to provide structural support. These strain concentrations can also be minimized by utilizing the largest possible curvature radii in the bridges 70 while maintaining feature widths that are proportional to the applied forces. Another way to minimize the strain concentrations is to minimize the maximum open area between the struts 60, loops 62 and bridges 70 in the tissue repair matrix in the expanded state.

These design characteristics are illustrated in FIG. 4. The largest radii curvature features in inventive tissue repair matrix are at the bridge-to-loop connections 76 which are non-symmetric with respect to the centers 64 of the strut connecting loop 62. In other words, the bridge-to-loop connection point centers 76 are off set from the center 64 of the loops 62 to which they are attached. The non-symmetric bridge connection point 76 is particularly advantageous for a tissue repair matrix having large expansion ratios because such sheets have extreme bending requirements and large elastic strains.

Nitinol can withstand extremely large amounts of elastic strain deformation, so the above features are well suited to a tissue repair matrix made from this alloy. This feature allows for maximum utilization of Nitinol or other material capabilities to enhance radial strength, improve tissue repair matrix strength uniformity, improves fatigue life by minimizing local strain levels and improves tissue repair matrix apposition in irregular organ wall shapes and curves.

Another design feature that improves the uniform expansion of the tissue repair matrix is the angle of the bridges that connect the adjacent elongated sections of the inventive tissue repair matrix. As the tissue repair matrix is transformed from its compressed state to its expanded state, strains are applied to the struts and loops. The forces of the expanding struts and loops are delivered to the bridge ends and alter the angle of the bridges with respect to the loops to which they are connected. As shown in FIGS. 1, 2 and 6, the angles of the bridges 70 connecting the first elongated strip 52(a) to the second elongated strip 52(b) and the third elongated strip 52(c) to the fourth elongated strip 52(d) are angled upwardly from left to right in an identical manner but the bridges connecting between the second elongated strip 52(b) and the third elongated strip 52(c) are angled downward from left to right in the opposite direction. This pattern of alternating bridge angles between the elongated strips would continue across a tissue repair matrix having additional elongated strips. This alternating bridge 70 slope pattern improves the rigidity of the tissue repair matrix 50 and minimizes any asymmetric movement or misalignment of the tissue repair matrix 50 within the patient. This symmetric deformation is particularly beneficial if the tissue repair matrix starts to shear in vivo.

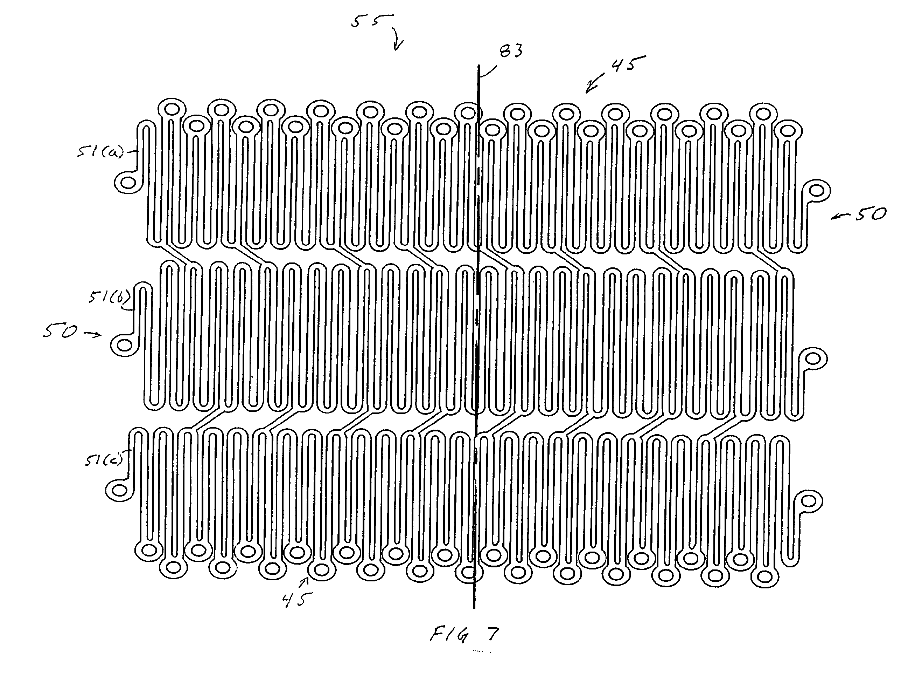

In an alternative embodiment illustrated in FIG. 7, the repair matrix 55 has elongated strips 51 (a)-51 (c) that run horizontally across the length of the repair matrix 55. The adjacent elongated strips 51(a)-51(c) include struts 60 and loops 62 that couple the adjacent struts 60. The adjacent elongated strips 51(a)-51(c) are coupled to each other with bridges 70. The elongated strips 51 (a)-51 (c) expand horizontally along the length of the medical sheet 55 rather than expanding across the width of the repair matrix as shown in FIGS. 1, 2, and 6. The ends of the elongated strips 52(a)-52(c) form the retractable sides 43 of the repair matrix 55. The edges of the repair matrix 55 that are formed by the upper side of the elongated strip 52(a) and the lower side of elongated strip 52(c) are the expandable sides 45 of the medical sheet 55. A longitudinal axis 83 extends between the retractable sides 43 of the repair matrix 55.

In addition to changing the direction of expansion, the alignment of the elongated strips will also influence the mechanical properties of the repair matrix in the expanded state. With reference to Fig. 6, the repair matrix are more elastic in the expanded state in a direction along the longitudinal direction L of the elongated strips 52(a) - 52(d). In contrast, the elongated strips 52(a) - 52(d) are less elastic across their widths W. Thus, the repair matrix shown in FIGS. 1 and 2 with vertically oriented elongated strips 52(a)-52(d) will be more vertically elastic in the expanded state. Similarly, the repair matrix shown in FIG. 7 has horizontally oriented elongated strips 51(a)-51(c) and will be more horizontally elastic in the expanded state.

According to the description herein, the inventive tissue repair matrix can be altered for many different implantation applications by changing the lengths and number of elongated sections. The inventive tissue repair matrix can be built for very specific applications including: lung repair, pleurodesis, hernia repair, skin grafting and other organ repair applications.

Although particular embodiments of the present invention have been shown and described, modification may be made to the device and/or method without departing from the scope of the present invention, which is defined by the appended claims.

## Claims

1. A method for manufacturing a tissue repair matrix (50) for implantation into a patient, comprising the steps:
a) vapor depositing a super elastic alloy thin film layer onto a substrate in a vacuum chamber;
b) separating the thin film layer from the substrate;
c) forming a pattern in the thin film layer comprising a plurality of elongated strips (52) wherein adjacent strips (52) are coupled by a plurality of bridges (70) and each strip (52) comprises a plurality of longitudinal struts (60) and a plurality of loops (62) connecting adjacent struts (60), wherein the patterned thin film layer has first and second expandable side edges, first and second retractable side edges and a longitudinal axis extending between the first and the second retractable side edges, the thin film layer having a first smaller area position for insertion into the patient, and a second larger area position for implantation into the patient; and
cutting a plurality of hooks (95) in the thin film layer having a first end attached to the thin film layer and a second pointed end (99) that is not attached to the thin film layer; and
bending the pointed end (99) of at least one of the hooks (95) away from the thin film layer so that the at least one hook (95) forms an angle with respect to the thin film layer when the thin film layer is in the second larger area position.

2. The method for manufacturing a tissue repair matrix according to claim 1 wherein the forming steps are performed by laser cutting through the thin film layer.

3. The method for manufacturing a tissue repair matrix according to claim 1 wherein the forming steps are performed by photo chemical etching the thin film layer.

4. The method for manufacturing a tissue repair matrix according to claim 1 further comprising the step:
forming a plurality of amorphic circles (91), wherein some of the amorphic circles (91) are attached to at least some of the plurality of loops (62).

5. The method for manufacturing a tissue repair matrix according to claim 4 further comprising the step:
forming holes (93, 97) in at least some of the plurality of amorphic circles (91).

6. The method for manufacturing a tissue repair matrix according to claim 4 further comprising the steps:
cutting at least some of the plurality of hooks (95) in some of the amorphic circles (91).

7. A tissue repair matrix (50) for implantation into a patient comprising:
a) a thin film layer of superelastic alloy having first and second expandable side edges, first and second retractable side edges and a longitudinal axis extending between the first and the second retractable side edges, the thin film layer having a first smaller area position for insertion into the patient, and a second larger area position for implantation into the patient;
b) a plurality of adjacent strips (52) extending parallel to each other along the longitudinal axis of the thin film layer, the strips (52) comprising a plurality of longitudinal struts (60) and a plurality of loops (62) connecting adjacent struts (60);
c) a plurality of bridges (70) connecting the adjacent strips (52) to one another at bridge-to-loop connection points, wherein the bridge-to-loop connection points (76) for each bridge (70) are separated angularly with respect to the longitudinal axis;
and
d) a plurality of hooks (95) that are cut into the thin film layer having a first end attached to the thin film layer and a second pointed end (99) that is not attached to the thin film layer, wherein the pointed end (99) of at least one of the hooks (95) is bent away from the thin film layer so that the at least one hook (95) forms an angle with respect to the thin film layer when the thin film layer is in the second larger area position.

8. The tissue repair matrix (50) according to claim 7 wherein the thin film layer is made from an alloy comprising from about 50.5 percent to about 60 percent Nickel and the remainder comprising Titanium.

9. The tissue repair matrix (50) according to claim 8 wherein the thin film layer has an A_{f} temperature between about 24° to about 37° Celsius.

10. A tissue repair matrix (50) according to claim 9, wherein the number of bridge-to-loop connection points is less than the total number of loops on the strips.

11. The tissue repair matrix (50) according to claim 7 or 10 further comprising:
a plurality of amorphic circles (91) that are attached to at least some of the plurality of loops (62).

12. The tissue repair matrix (50) according to claim 7 or 10 wherein at least some of the plurality of hooks (95) are formed in some of the amorphic circles (91).

13. The tissue repair matrix (50) according to claim 10 wherein at least some of the plurality of hooks (95) are not bent away from the thin film member when the thin film member is in the first smaller surface area position.

14. The tissue repair matrix (50) according to claim 11 wherein at least some of the plurality of amorphic circles (91) have concentric holes (93).

15. The tissue repair matrix (50) according to claim 11 wherein some of the plurality of amorphic circles (91) are attached to the plurality of bridges (70).

16. The tissue repair matrix (50) according to claim 11 wherein some of the plurality of amorphic circles (91) are attached to at least some of the plurality of struts (60).

## Patentansprüche

1. Verfahren zum Herstellen einer Gewebereparaturmatrix (50) zur Implantation in einen Patienten, wobei das Verfahren die folgenden Schritte umfasst:
a) Dampfabscheiden einer superelastischen Legierungsdünnfilmschicht auf ein Substrat in einer Vakuumkammer;
b) Trennen der Dünnfilmschicht von dem Substrat;
c) Bilden eines Musters in der Dünnfilmschicht, welches mehrere längliche Streifen (52) umfasst, wobei benachbarte Streifen (52) durch mehrere Brücken (70) gekoppelt sind und jeder Streifen (52) mehrere longitudinale Streben (60) und mehrere Schlaufen (62), welche benachbarte Streben (60) verbinden, umfasst, wobei die mit Muster versehene Dünnfilmschicht eine erste und eine zweite ausdehnbare Seitenkante, eine erste und eine zweite zurückziehbare Seitenkante und eine Längsache, welche sich zwischen der ersten und der zweiten zurückziehbaren Seitenkante erstreckt, aufweist, wobei die Dünnfilmschicht eine erste Position kleinerer Fläche zum Einführen in den Patienten und eine zweite Position größerer Fläche zur Implantation in den Patienten aufweist; und Schneiden mehrer Haken (95) in die Dünnfilmschicht, welche ein erstes Ende, welches an der Dünnfilmschicht angebracht ist, und ein zweites zugespitztes Ende (99), welches nicht an der Dünnfilmschicht angebracht ist, aufweisen, und
Wegbiegen des zugespitzten Endes (99) zumindest eines der Haken (95) von der Dünnfilmschicht, so dass der zumindest eine Haken (95) bezüglich der Dünnfilmschicht einen Winkel bildet, wenn die Dünnfilmschicht in der zweiten Position größerer Fläche ist.

2. Verfahren zum Herstellen einer Gewebereparaturmatrix gemäß Anspruch 1, wobei die Schritte des Bildens durch ein Laserschneiden durch die Dünnfilmschicht durchgeführt werden.

3. Verfahren zum Herstellen einer Gewebereparaturmatrix gemäß Anspruch 1, wobei die Schritte des Bildens durch ein fotochemisches Ätzen der Dünnfilmschicht durchgeführt werden.

4. Verfahren zum Herstellen einer Gewebereparaturmatrix gemäß Anspruch 1, welches weiter den folgenden Schritt umfasst:
Bilden mehrerer amorpher Ringe (91), wobei einige der amorphen Ringe (91) an zumindest einigen der mehreren Schlaufen (62) angebracht sind.

5. Verfahren zum Herstellen einer Gewebereparaturmatrix gemäß Anspruch 4, welches weiter den folgenden Schritt umfasst:
Bilden von Löchern (93, 97) in zumindest einigen der mehreren amorphen Ringe (91).

6. Verfahren zum Herstellen einer Gewebereparaturmatrix gemäß Anspruch 4, welches weiter den folgenden Schritt umfasst:
Schneiden zumindest einiger der mehreren Haken (95) in einigen der amorphen Ringe (91).

7. Gewebereparaturmatrix (50) zur Implantation in einen Patienten, wobei die Matrix Folgendes umfasst:
a) eine Dünnfilmschicht aus superelastischer Legierung mit einer ersten und einer zweiten ausdehnbaren Seitenkante, einer ersten und einer zweiten zurückziehbaren Seitenkante und einer Längsachse, welche sich zwischen der ersten und der zweiten zurückziehbaren Seitenkante erstreckt, wobei die Dünnfilmschicht eine erste Position kleinerer Fläche zum Einführen in den Patienten und eine zweite Position größerer Fläche zur Implantation in den Patienten aufweist;
b) mehrere benachbarte Streifen (52), welche sich parallel zueinander entlang der Längsachse der Dünnfilmschicht erstrecken, wobei die Streifen (52) mehrere longitudinale Streben (60) und mehrere Schlaufen (62), welche benachbarte Streben (60) verbinden, umfassen;
c) mehrere Brücken (70), welche die benachbarten Streifen (52) miteinander an Brücke-zu-Schlaufe-Verbindungspunkten verbinden, wobei die Brücke-zu-Schlaufe-Verbindungspunkte (76) für jede Brücke (70) bezüglich der Längsachse winkelbeabstandet sind; und
d) mehrere Haken (95), welche in die Dünnfilmschicht geschnitten sind und ein erstes, an der Dünnfilmschicht angebrachtes Ende und ein zweites zugespitztes Ende (90), welches nicht an der Dünnfilmschicht angebracht ist, aufweisen, wobei das zugespitzte Ende (99) zumindest eines der Haken (95) von der Dünnfilmschicht weggebogen ist, so dass der zumindest eine Haken (95) bezüglich der Dünnfilmschicht einen Winkel bildet, wenn die Dünnfilmschicht in der zweiten Position größerer Fläche ist.

8. Gewebereparaturmatrix (50) gemäß Anspruch 7, wobei die Dünnfilmschicht aus einer Legierung, welche von etwa 50,5% bis etwa 60% Nickel umfasst, und wobei der Rest Titan umfasst, hergestellt ist.

9. Gewebereparaturmatrix (50) gemäß Anspruch 8, wobei die Dünnfilmschicht eine A_{f}-Temperatur zwischen etwa 24° bis etwa 37° Celsius aufweist.

10. Gewebereparaturmatrix (50) gemäß Anspruch 9, wobei die Anzahl von Brücke-zu-Schlaufe-Verbindungspunkten kleiner als die Gesamtzahl von Schlaufen auf den Streifen ist.

11. Gewebereparaturmatrix (50) gemäß Anspruch 7 oder 10, wobei die Matrix weiter Folgendes umfasst:
mehrere amorphe Ringe (91), welche an zumindest einigen der mehreren Schlaufen (62) angebracht sind.

12. Gewebereparaturmatrix (50) gemäß Anspruch 7 oder 10, wobei zumindest einige der mehreren Haken (95) in einigen der amorphen Ringe (91) gebildet sind.

13. Gewebereparaturmatrix (50) gemäß Anspruch 10, wobei zumindest einige der mehreren Haken (95) nicht von dem Dünnfilmelement weggebogen sind, wenn das Dünnfilmelement in der ersten Position kleineren Oberflächeninhalts ist.

14. Gewebereparaturmatrix (50) gemäß Anspruch 11, wobei zumindest einige der mehreren amorphen Ringe (91) konzentrische Löcher (93) aufweisen.

15. Gewebereparaturmatrix (50) gemäß Anspruch 11, wobei einige der mehreren amorphen Ringe (91) an den mehreren Brücken (70) angebracht sind.

16. Gewebereparaturmatrix (50) gemäß Anspruch 11, wobei einige der mehreren amorphen Ringe (91) an zumindest einigen der mehreren Streben (60) angebracht sind.

## Revendications

1. Procédé pour fabriquer une matrice de réparation tissulaire (50) destinée à être implantée dans un patient, comprenant les étapes consistant à :
a) déposer en phase vapeur une couche de film fin en alliage super élastique sur un substrat dans une chambre sous vide ;
b) séparer la couche de film fin du substrat ;
c) former un motif dans la couche de film fin comprenant une pluralité de bandes allongées (52), dans lequel les bandes (52) adjacentes sont couplées par une pluralité de ponts (70) et chaque bande (52) comprend une pluralité d'entretoises longitudinales (60) et une pluralité de boucles (62) raccordant les entretoises (60) adjacentes, dans lequel la couche de film fin à motifs a des premier et deuxième bords latéraux expansibles, des premier et deuxième bords latéraux rétractables et un axe longitudinal s'étendant entre les premier et deuxième bords latéraux rétractables, la couche de film fin ayant une première position de plus petite surface pour l'insertion dans le patient, et une deuxième position de plus grande surface pour l'implantation dans le patient ; et
✔ couper une pluralité de crochets (95) dans la couche de film fin ayant une première extrémité fixée à la couche de film fin et une deuxième extrémité pointue (99) qui n'est pas fixée à la couche de film fin ; et
✔ plier l'extrémité pointue (99) d'au moins l'un des crochets (95) à distance de la couche de film fin de sorte que le au moins un crochet (95) forme un angle par rapport à la couche de film fin lorsque la couche de film fin est dans la deuxième position de plus grande surface.

2. Procédé pour fabriquer une matrice de réparation tissulaire selon la revendication 1, dans lequel les étapes de formation sont réalisées en découpant au laser la couche de film fin.

3. Procédé pour fabriquer une matrice de réparation tissulaire selon la revendication 1, dans lequel les étapes de formation sont réalisées en gravant la couche de film fin par procédé photochimique.

4. Procédé pour fabriquer une matrice de réparation tissulaire selon la revendication 1, comprenant en outre l'étape consistant à :
✔ former une pluralité de cercles amorphes (91), dans lequel certains des cercles amorphes (91) sont fixés sur au moins certaines de la pluralité de boucles (62).

5. Procédé pour fabriquer une matrice de réparation tissulaire selon la revendication 4, comprenant en outre l'étape consistant à :
✔ former des trous (93, 97) dans au moins certains de la pluralité de cercles amorphes (91) .

6. Procédé pour fabriquer une matrice de réparation tissulaire selon la revendication 4, comprenant en outre les étapes consistant à :
✔ couper au moins certains de la pluralité de crochets (95) dans certains des cercles amorphes (91).

7. Matrice de réparation tissulaire (50) destinée à être implantée dans un patient comprenant :
a) une couche de film fin en alliage super élastique ayant des premier et deuxième bords latéraux expansibles, des premier et deuxième bords latéraux rétractables et un axe longitudinal s'étendant entre les premier et deuxième bords latéraux rétractables, la couche de film fin ayant une première position de plus petite surface pour l'insertion dans le patient et une deuxième position de plus grande surface pour l'implantation dans le patient ;
b) une pluralité de bandes (52) adjacentes s'étendant parallèlement entre elles le long de l'axe longitudinal de la couche de film fin, les bandes (52) comprenant une pluralité d'entretoises longitudinales (60) et une pluralité de boucles (62) raccordant les entretoises (60) adjacentes ;
c) une pluralité de ponts (70) raccordant les bandes (52) adjacentes les unes aux autres au niveau de points de raccordement de pont à boucle, dans laquelle les points de raccordement de pont à boucle (76) pour chaque pont (70) sont séparés de manière angulaire par rapport à l'axe longitudinal ; et
d) une pluralité de crochets (95) qui sont coupés dans la couche de film fin ayant une première extrémité fixée à la couche de film fin et une deuxième extrémité pointue (99) qui n'est pas fixée à la couche de film fin, dans laquelle l'extrémité pointue (99) d'au moins l'un des crochets (95) est pliée à distance de la couche de film fin de sorte que le au moins un crochet (95) forme un angle par rapport à la couche de film fin lorsque la couche de film fin est dans la deuxième position de plus grande surface.

8. Matrice de réparation tissulaire (50) selon la revendication 7, dans laquelle la couche de film fin est réalisée à partir d'un alliage comprenant environ 50,5 pour cent à environ 60 pour cent de nickel et le reste comprenant du titane.

9. Matrice de réparation tissulaire (50) selon la revendication 8, dans laquelle la couche de film fin a une température A_{f} comprise entre environ 24° et environ 37°C.

10. Matrice de réparation tissulaire (50) selon la revendication 9, dans laquelle le nombre de points de raccordement de pont à boucle est inférieur au nombre total de boucles sur les bandes.

11. Matrice de réparation tissulaire (50) selon la revendication 7 ou 10, comprenant en outre :
✔ une pluralité de cercles amorphes (91) qui sont fixés à au moins certaines de la pluralité de boucles (62).

12. Matrice de réparation tissulaire (50) selon la revendication 7 ou 10, dans laquelle au moins certains de la pluralité de crochets (95) sont formés dans certains des cercles amorphes (91).

13. Matrice de réparation tissulaire (50) selon la revendication 10, dans laquelle au moins certains de la pluralité de crochets (95) ne sont pas pliés à distance de l'élément de film fin lorsque l'élément de film fin est dans la première position de plus petite surface.

14. Matrice de réparation tissulaire (50) selon la revendication 11, dans laquelle au moins certains de la pluralité de cercles amorphes (91) ont des cercles concentriques (93).

15. Matrice de réparation tissulaire (50) selon la revendication 11, dans laquelle certains de la pluralité de cercles amorphes (91) sont fixés à la pluralité de ponts (70).

16. Matrice de réparation tissulaire (50) selon la revendication 11, dans laquelle certains de la pluralité de cercles amorphes (91) sont fixés sur au moins certaines de la pluralité d'entretoises (60).
